# EUROPÄISCHE PATENTSCHRIFT

(11) **EP 0 693 487 B1**
(45) Veröffentlichungstag und Bekanntmachung des Hinweises auf die Patenterteilung: **03.09.1997**
(21) Anmeldenummer: 95110782.0
(22) Anmeldetag: 11.07.1995
(51) Int. Cl.: C07D 317/36, A61K 6/083

(54) **Di(meth)acrylate mit cyclischen Carbonatgruppen**
Di(meth)acrylates with cyclic carbonate groups
Di(méth)acrylates avec des groupes carbonates cycliques

(30) Priorität: 22.07.1994 DE 4426129
(43) Veröffentlichungstag der Anmeldung: 24.01.1996
(73) Patentinhaber: Heraeus Kulzer GmbH & Co.KG, 63450 Hanau (DE)
(72) Erfinder: Podszun, Wolfgang, Dr., D-51061 Köln (DE); Schäpers, Klaus, Dr., D-50259 Pulheim (DE); Finger, Werner, Prof.Dr., D-41469 Neuss (DE); Heiliger, Ludger, Dr., D-51373 Leverkusen (DE); Casser, Carl, Dr., D-51061 Köln (DE)
(74) Vertreter: Kühn, Hans-Christian

(56) Entgegenhaltungen:
- US-A- 3 774 305
- DENTAL MATERIALS JOURNAL, Bd.4, Nr.1, 1985 Seiten 33 - 39 TADAO FUKUSHIMA ET AL. 'Application of Functional Monomers for Dental Use (Part-9). Syntheses of Sccinoxy Methacrylates and Their Adhesion to Polished and Etched Tooth Surfaces'

## Beschreibung

Die Erfindung betrifft Diacrylate und Dimethacrylate mit cyclischen Carbonatgruppen, sowie ihre Verwendung in Dentalwerkstoffen.

Di(meth)acrylate werden auf dem Dentalgebiet z.B. als Bestandteil von Kunststofffüllungsmaterialien, Zahnlacken, Versieglern, Befestigungsmaterialien und Verblendmaterialien angewendet.

Ein besonders häufig verwendetes Monomer ist das sogenannte BisGMA der Formel (I).

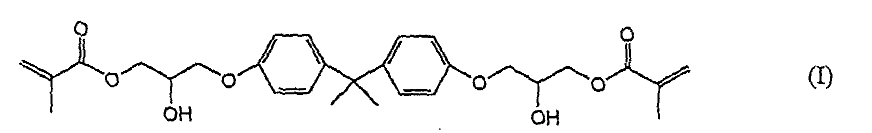

Werkstoffe aus BisGMA weisen ein günstiges Niveau der mechanischen Eigenschaften auf.

In Dental Materials Journal 4(1), 33 - 39, 1985,werden die Darstellung einiger Succinyloxyalkylmethacrylate und ihre Polymerisation beschrieben. Die Möglichkeit, die Haftung von Dentalmaterialien auf Kunststoff-Basis an der Zahnsubstanz - Dentin und Schmelz - mit Hilfe der Succinyloxyalkylmethacrylate zu verbessern, wird untersucht.

Eine bevorzugte Aushärtungsmethode für viele Anwendungen ist die Photopolymerisation unter Bestrahlung mit sichtbarem Licht. Für die Aushärtung von Zahnfüllungsmaterialien beträgt die notwendige Belichtungsdauer üblicher Weise 15 bis 60 s. Die Aushärtungstiefen betragen im allgemeinen einige mm und sind bei stark pigmentierten Füllungsmaterialien deutlich niedriger als bei transparenten Füllungsmaterialien. Bei tiefen Kavitäten muß der Zahnarzt die Füllung schichtweise aushärten.

Ein Mangel aller bisher bekannten photopolymeriserbaren Dentalmaterialien besteht darin, daß sie in Kontakt mit Luftsauerstoff an der freien Oberfläche nicht aushärten. Dieses als Polymerisationsinhibition bekannte Phänomen erfordert besondere Maßnahmen zum Ausschluß des Luftsauerstoffes. Eine gut wirksame Maßnahme bei glatten Oberflächen ist die Abdeckung mit einer transparenten sauerstoffundurchlässigen Folie. Für geometrisch kompliziertere Oberflächen wurden Schutzüberzüge (z.B. aus Polyvinylalkohol), die aus Lösung aufgetragen werden, als Sauerstoffbarriere vorgeschlagen, doch ist diese Methode nicht nur aufwendig sondern auch weniger wirksam. In vielen Fällen behilft sich der Zahnarzt in der Weise, daß er einen Überschuß an Dentalmaterial anwendet und den nicht polymerisierten Teil nach der Belichtung in einem Nachbearbeitungsschritt entfernt. Auch diese Methode birgt gravierende Nachteile: Die Gestaltung der Oberflächengeometrie ist nur sehr ungenau möglich. Die Grenze zwischen nicht oder unzureichend polymerisiertem Material einerseits und vollständig ausgehärtetem Material andererseits ist nicht erkennbar, so daß die Gefahr besteht, daß bei der Nachbearbeitung eine nicht optimal ausgehärtete Oberfläche erhalten wird.

Die der vorliegenden Erfindung zugrundeliegende Aufgabe ist die Bereitstellung neuer Monomere mit erhöhter Polymerisationsgeschwindigkeit und verringerter Empfindlichkeit gegenüber Polymerisationshemmung durch Sauerstoff, insbesondere für Anwendungen auf dem Dentalgebiet.

Die Ausgabe wird erfindungsgemäß durch Monomere mit cyclischen Carbonatgruppen gemäß Formel (II) gelöst. 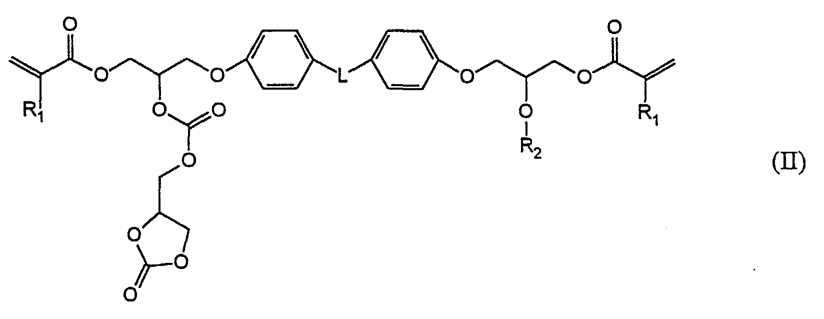 worin unabhängig voneinander
- R₁: unabhängig voneinander für Wasserstoff oder Methyl,
- R₂: für Wasserstoff oder 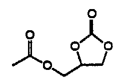 und
- L: für eine direkte C-C-Bindung, -O-, -S-, -SO₂-, -CO-, einen C₁-C₁₅-Alkylenrest, der mit Alkyl, Hydroxyl oder Halogen substituiert sein kann, den Cyclohexylidenrest oder den 3,3,5-Trimethylcyclohexylidenrest steht.

Nachfolgend seien bevorzugte Beispiele für L genannt:

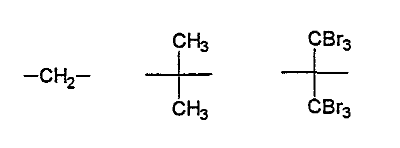

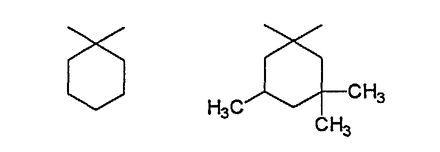

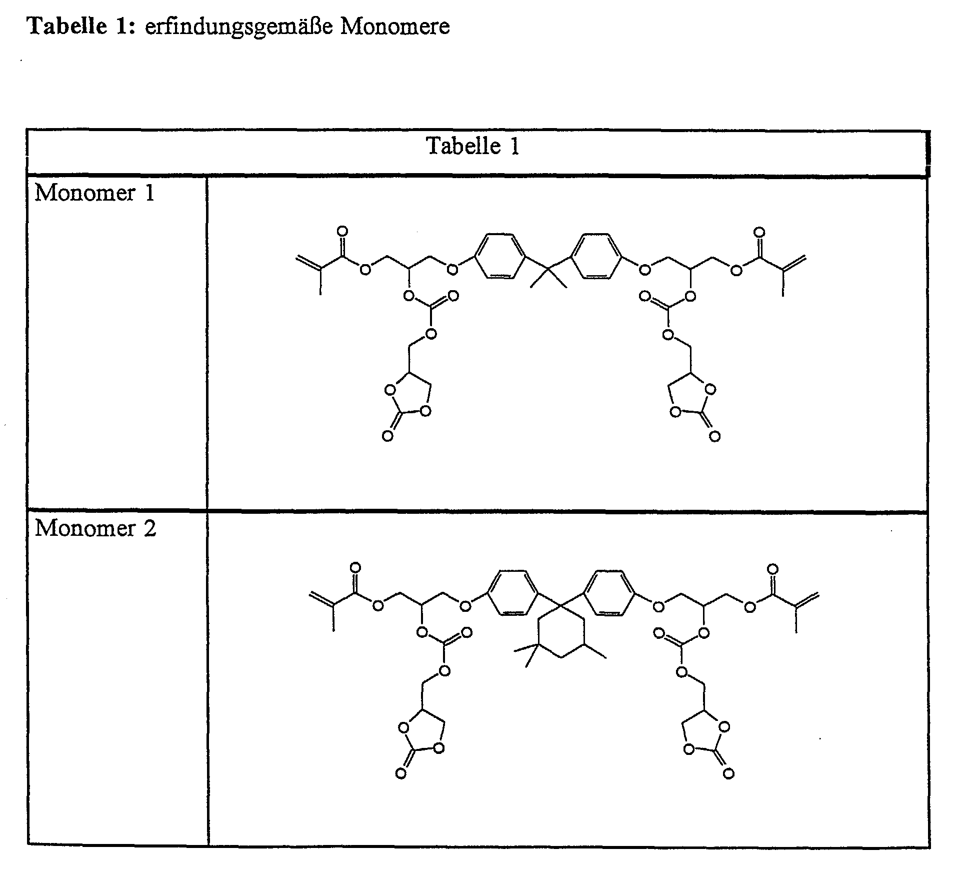

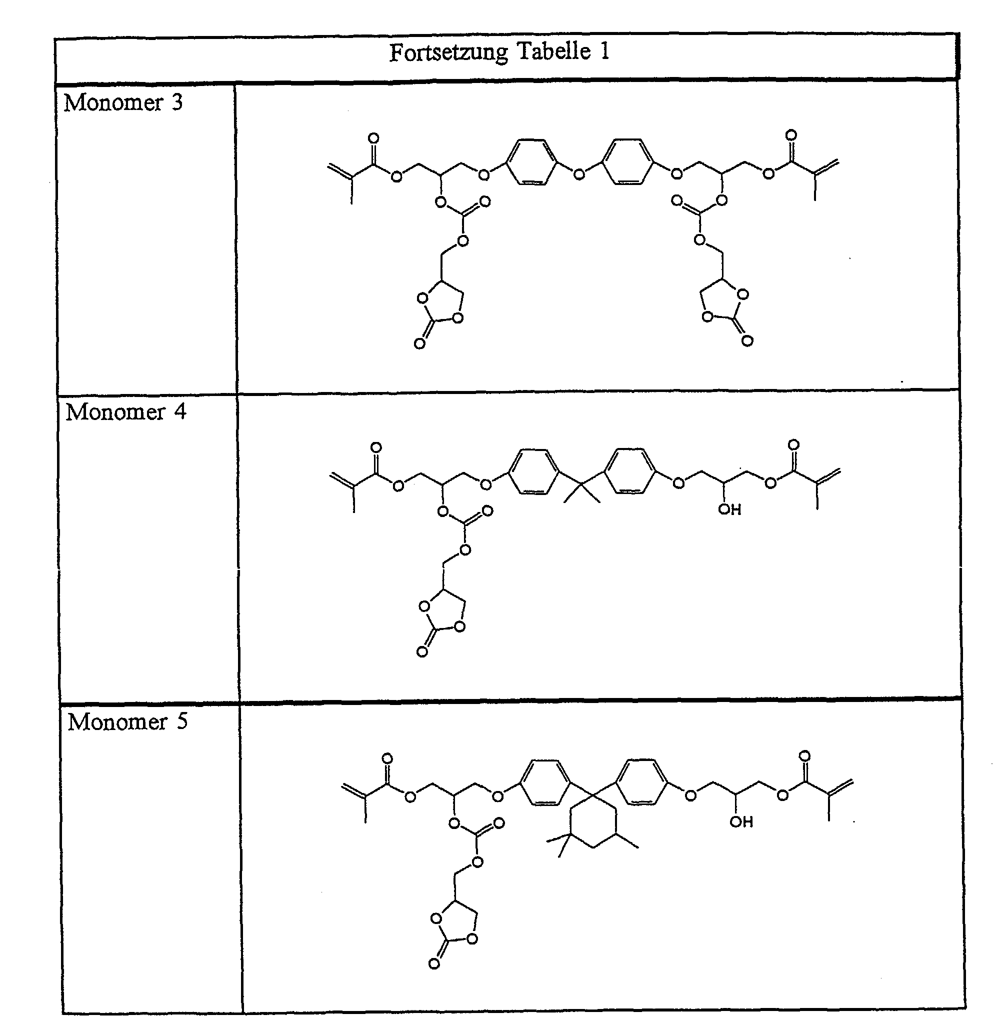

Die Synthese der erfindungsgemäßen (Meth)acrylate mit cyclischen Carbonatgruppen erfolgt zweckmäßiger Weise durch Umsetzung der zugrundeliegenden Hydroxylverbindung mit dem Chlorameisensäureester gemäß Formel (III).

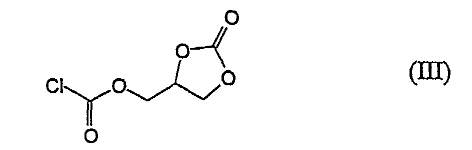

Der Chlorameisensäureester gemäß Formel (III) kann durch Phosgenierung von Glycerin hergestellt werden. Dieser Syntheseschritt wird in US 2 446 145 ausführlich beschrieben.

Für die Anwendung der erfindungsgemäßen Monomere in polymerisierbaren dentalen Restaurierungsmaterialien können die erfindungsgemäßen (Meth)acrylsäureester mit an sich bekannten Monomeren gemischt werden. Als Mischungskomponente sind beispielsweise das BisGMA nach Formel (I) und Urethanmethacrylate, die durch Umsetzung von Diisocyanaten mit Hydroxyethylmethacrylat zugänglich sind, besonders gut geeignet.

Die Viskosität der erfindungsgemäßen Monomeren ist niedriger als die der herkömmlichen Verbindungen (z.B. BisGMA), so daß erfindungsgemäße Verbindungen der Formel (II) auch unverdünnt, d.h. in reiner Form, angewendet werden können. Falls eine noch niedrigere Viskosität gewünscht wird, können den erfindungsgemäßen Monomeren Comonomere niedriger Viskosität als Reaktivverdünner bzw. Lösungsmittel zugemischt werden. Die erfindungsgemäßen Verbindungen werden dann in Mischung mit Comonomeren in einem Anteil von minimal 10 Gew.-%, bevorzugt von 20 Gew.-%, eingesetzt.

Beispielsweise seien die folgenden Comonomere genannt: Glycerindimethacrylat, Triethylenglykoldimethacrylat (TEGDMA), Tetraethylenglykoldimethacrylat, 1,12-Dodecandioldimethacrylat, 1,6-Hexandioldimethacrylat, Diethylenglykoldimethacrylat, 2,2-Bis-[p-(2'-hydroxy-3'-methacryloyloxypropoxy)-phenyl]-propan, 2,2-Bis-[p-(2'-methacryloyloxyethoxy)-phenyl]-propan, Trimethylol-propan-tri-(meth)acrylat, Bis-(meth)-acryloyloxyethoxymethyl-tricyclo[5,2,1,0^{2,6}]-decan (DE-A-29 31 925 und DE-A-29 31 926).

Insbesondere bevorzugt werden Comonomere, die bei 13 mbar einen Siedepunkt über 100°C besitzen.

Die erfindungsgemäßen (Meth)acrylsäureester können, gegebenenfalls in Mischung mit den genannten Comonomeren, mit an sich bekannten Methoden zu vernetzten Polymerisaten ausgehärtet werden (Am. Chem. Soc., Symp. Ser. 212, 359-371 (1983)). Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind: Dibenzoylperoxid, Dilauroylperoxid und Di-4-chlorbenzoylperoxid.

Als tertiäre aromatische Amine seien beispielsweise N,N-Dimethyl-p-toluidin, Bis-(2-hydroxyethyl)-p-toluidin, Bis-(2-hydroxyethyl)-3,5-dimethylanilin und N-Methyl-N-(2-methylcarbamoyloxypropyl)-3,5-dimethylanilin genannt. Die Konzentration des Peroxids bzw. des Amins werden vorteilhaft so gewählt, daß sie 0,1 bis 5 Gew.-%, bevorzugt 0,5 bis 3 Gew.-%, bezogen auf die Monomermischung betragen.

Die peroxid- bzw. aminhaltigen Monomermischungen werden bis zur Anwendung getrennt gelagert.

Die erfindungsgemäßen Monomeren können auch durch Bestrahlung mit UV-Licht oder sichtbarem Licht zur Polymerisation gebracht werden. Als Initiatoren für die photoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Aktivatoren, wie N,N-Dimethylaminoethylmethacrylat, Triethanolamin, 4-N,N-Dimethylaminobenzolsulfon-säurediallylamid. Die Durchführung der Photopolymerisation ist beispielsweise in der DE-A-3 135 115 beschrieben.

Neben den oben beschriebenen Initiatoren können den erfindungsgemäßen Monomere an sich für diesen Einsatzzweck bekannte Lichtschutzmittel und Stabilisatoren zugesetzt werden.

Lichtschutzmittel sind beispielsweise in "Gächter, Müller, Taschenbuch der Kunststoff-Additive, 3. Ausgabe, Carl Hanser Verlag" beschrieben. Beispielsweise seien die folgenden Lichtschutzmittel genannt: Cyasorb UV 9®, Tinuvin P®, Tinuvin 770®, Tinuvin 622®, Tinuvin 765®.

Stabilisatoren sind beispielsweise in "Ullmanns Encyclopädie der technischen Chemie, 4. Auflage, Band 8, Seite 19-45" beschrieben. Beispielsweise seien die folgenden Stabilisatoren genannt: 2,6-Di-tert.-butylphenol, 2,6-Di-tert.-butyl-4-methylphenol, 2,6-Di-octadecyl-4-methyl-phenol, 1,1'-Methylen-bis(naphthol-2).

Die Lichtschutzmittel und die Stabilisatoren können jeweils in einer Menge von 0,01 bis 0,5 Gew.-Teilen, bezogen auf 100 Gew.-Teile der Monomermischung, eingesetzt werden.

Die Monomermischungen können mit oder ohne Zusatz von Füllstoffen auch als Beschichtungsmittel (Zahnlacke) und als Adhäsive (Schmelz und Dentin) eingesetzt werden.

Bei der Verwendung als Zahnfüllmassen oder Befestigungsmaterialien setzt man den erhaltenen Monomermischungen im allgemeinen Füllstoffe zu. Um einen hohen Füllgrad erreichen zu können, sind Monomermischungen, die eine Viskosität im Bereich von 60 bis 10.000 mPa·s besitzen, besonders vorteilhaft.

Vorzugsweise mischt man den erfindungsgemäßen Monomeren anorganische Füllstoffe zu. Beispielsweise seien Bergkristall, Kristobalit, Quarzglas, hochdisperse Kieselsäure, Aluminiumoxid und Glaskeramiken, beispielsweise Zirkon enthaltende Glaskeramiken genannt. Die anorganischen Füllstoffe werden zur Verbesserung des Verbunds zur Polymermatrix des Polymethacrylats, vorzugsweise mit einem Haftvermittler, vorbehandelt. Die Haftvermittlung kann beispielsweise durch eine Behandlung mit Organosiliciumverbindungen erreicht werden (Progress in Organic Coatings 11, 297-308 (1983). Bevorzugt wird 3-Methacryloyloxypropyl-trimethoxysilan eingesetzt. Die Füllstoffe für die erfindungsgemäßen Zahnfüllmassen weisen im allgemeinen einen mittleren Teilchendurchmesser von 0,01 bis 100 µm, vorzugsweise von 0,03 bis 50 µm, besonders bevorzugt von 0,03 bis 5 µm auf. Es kann auch vorteilhaft sein, mehrere Füllstoffe nebeneinander einzusetzen, die einen voneinander verschiedenen mittleren Teilchendurchmesser und/oder einen unterschiedlichen Silangehalt besitzen.

Der Füllstoffanteil in der Zahnfüll- wie in der Befestigungsmasse beträgt im allgemeinen 5 bis 85 Gew.-%, vorzugsweise 50 bis 80 Gew.-%.

Für die Herstellung der Restaurierungsmassen werden die Komponenten unter Verwendung handelsüblicher Knetmaschinen vermischt.

Der Anteil der erfindungsgemäßen (Meth)acrylate in den Restaurierungsmassen beträgt im allgemeinen 5 bis 60 Gew.-%,bezogen auf die Restaurierungsmasse.

Zahnlacke, Adhäsive und Restaurationsmaterialien, die die erfindungsgemäßen Monomere enthalten, zeigen eine äußerst hohe Polymerisationsgeschwindigkeit, wobei die Polymerisation nur wenig durch Luftsauerstoff gestört wird. Bei der Photopolymerisation werden große Aushärtungstiefen erreicht.

Zur Prüfung der Empfindlichkeit erfindungsgemäßer Monomere gegenüber Polymerisationsinhibition durch Luftsauerstoff bieten sich grundsätzlich zwei Methoden an:
1. Bestimmung der Schichtdicke unpolymerisierten Resins an der freien Oberfläche dünner, scheibenförmiger Proben zwischen zwei Glasplatten. Aufgrund der unterschiedlichen Brechungsindices von polymerisiertem und unpolymerisiertem Resin, läßt sich im Durchlichtmikroskop eine Trennlinie erkennen. Weitere Einzelheiten zu dieser Methode sind in der Literatur beschrieben: Polymerisationsinhibition durch Sauerstoff bei Kompositfüllungsmaterialien und Schmelzversieglern, W. Finger und K. Dreyer Jörgensen, Schweiz. Mschr. Zahnheilkunde, 86, 812-824, (1976).
2. Bestimmung der unpolymerisierten Schichtdicke durch Messung der Probenoberfläche vor und nach Auflösung der Monomerschicht in einem geeigneten Lösungsmittel.

Die Polymerisations-Inhibitionsempfindlichkeit wurde an lichtaktivierten Monomeren wie 1 und 4 aus Tabelle 1 im Vergleich zu einer dentalüblichen Monomermischung (BisGMA-TEGDMA 63:38 % w/w) nach dem o. g. Verfahren 2 geprüft, da bei transparenten ungefüllten Systemen die Randbegrenzung des nicht polymerisierten Materials nach Verfahren 1 aufgrund von Meniskusbildung nur recht ungenau festgelegt werden kann.

### Beispiele

### Beispiel 1, Herstellung von Monomer 1 aus Tabelle 1

In 500g Chloroform wurden unter Stickstoffatmosphäre 102.52 g BisGMA (Formel (I)), 44.48 g Triethylamin und 0.16 g 2,6-Di-tert.-butylkresol (Stabilisator) gelöst und auf O°C gekühlt. Danach wurden 72.22 g Chlorameisensäureester der Formel (III), gelöst in 100 g Chloroform langsam zudosiert und der Ansatz 10 h bei Raumtemperatur nachgerührt. Der entstandene Niederschlag wurde abfiltriert und das Filtrat auf das doppelte Volumen Wasser gegossen. Die organische Phase wurde abgetrennt, 2 mal mit 0,1 n Salzsäure, dann mit Natriumhydrogencarbonatlösung ausgeschüttelt, schließlich mit Wasser gewaschen und über Natriumsulfat getrocknet. Nach Filtration und Abdampfen des Lösemittels verbleiben 120,8 g Monomer 1 aus Tabelle 1.
IR [cm⁻¹]: 1820 (cycl. Carbonat); 1760 (Carbonat); 1725 (Ester); 1645 (Methacryl).

### Beispiel 2, Herstellung von Monomer 4 aus Tabelle 1

Das Versuch wurde ausgeführt wie in Beispiel 1 beschrieben, mit der Änderung, daß 22.24 g Triethylamin und 36.11 g Chlorameisensäureester der Formel (III) eingesetzt wurden .
IR [cm⁻¹]: 1820 (cycl. Carbonat); 1760 (Carbonat); 1725 (Ester); 1640, 1620 (Methacryl).

### Beispiel 3, Überprüfung der Photoreaktivität der Monomeren mit Hilfe der Photo-DSC

Die folgenden Bestandteile wurden intensiv durchmischt:

| | |
|---|---|
| 5.0 g | Monomer |
| 100 mg | Campherchinon |
| 250mg | p-Dimethylaminobenzolsulfonsäure-N,N-diallylamid |

Campherchinon und p-Dimethylaminobenzolsulfonsäure-N,N-diallylamid bilden das Photoinitiatorsystem.

Die Proben wurden bei 30°C in einer DSC-Apperatur (Differential Scanning Calorimetry) mit einer Halogenlampe (75 W) mit Wärmeschutzfilter bestrahlt. Der Wärmefluß wurde unter Bestrahlung als Funktion der Zeit registriert. Als Referenz wurden Proben gleicher Zusammensetzung ohne Photoinitiator eingesetzt. Während des Versuchs wurde mit Stickstoff gespült. Für die Auswertung wurde als Maß für die Reaktionsgeschwindigkeit der Wert t-max ermittelt. t-max ist die Zeit von Bestrahlungsbeginn bis zum Erreichen des Maximum der Reaktion (maximaler Wärmefluß).
Je kleiner t-max ist, um so größer ist die Photoreaktivität.

| Monomer | t-max [min] |
|---|---|
| aus Beispiel 1 | 0,60 |
| aus Beispiel 2 | 0.45 |
| Bis-GMA-TEGDMA 62:38 % w/w (Vergleich) | 1.70 |

### Beispiel 4, Untersuchung der Empfindlichkeit gegenüber Polymerisationsinhibition durch Luftsauerstoff

Zur Bestimmung der Schichtdicke unpolymerisierten Monomers an der freien Oberfläche (normale Umgebungsatmosphäre) wurden zylindrische Metallformen (Ø = 5 mm, h = 2 mm) mit lichtaktiviertem Monomer (Aktivierung wie in Beispiel 3) aufgefüllt und 20 Sekunden lang mit einer handelsüblichen Polymerisationseinheit (Translux CL, Kulzer GmbH) im Abstand von ca. 2 mm belichtet. Unmittelbar nach der Polymerisation wurden im Auflichtmikroskop nach der Schärfentiefemethode an jeweils 7 durch die Kreuztischkoordinaten definierten Meßpunkten (x, y) auf einer Geraden, unter Einschluß von Referenzpunkten auf der freien Metalloberfläche, die Höhenkoordinaten (z-Werte) bestimmt. Die Proben wurden dann zur Entfernung der nicht polymersierten Oberflächenschicht unter Verwendung einer mittelharten Bürste mit Ethanol abgewaschen. Die Form wurde anschließend wieder in die Ausgangsposition auf dem Mikroskopkreuztisch montiert. Nach Anfahren der ursprünglichen x/y-Koordinaten wurden erneut die z-Werte bestimmt. Der Höhendifferenzwert vor und nach Abwaschen mit Alkohol entspricht der Monomerschichtdicke, die aufgrund der Sauerstoff-inhibition der Polymerisation an der Oberfläche verblieben war.

Die Monomerschichtdicken wurden an fünf Proben bestimmt und sind als Mittelwerte und Standardabweichungen in der folgenden Tabelle zusammengestellt:

| Monomer | Schichtdicke des unpolymerisierten Monomers [µm] |
|---|---|
| aus Beispiel 1 | 0.3 ± 0.4 |
| aus Beispiel 2 | 0.8 ± 0.2 |
| Bis-GMA-TEGDMA 62:38 % w/w (Vergleich) | 11.0 ± 1.4 |

Nach dem Abbürsten mit Ethanol zeigten die Vergleichsproben ausgeprägte Abriebsspuren, während Bürstenabriebsspuren der Proben aus den Monomeren der Beispiele 1 und 2 bei 200-facher Vergrößerung im Auflichtmikroskop kaum zu erkennen waren. Diese Beobachtung belegt ebenfalls eine bessere oberflächliche Polymerisation der Proben aus den Monomeren der Beispiele 1 und 2 im Vergleich zur Referenzprobe.

## Patentansprüche

1. Verbindungen der Formel (II) 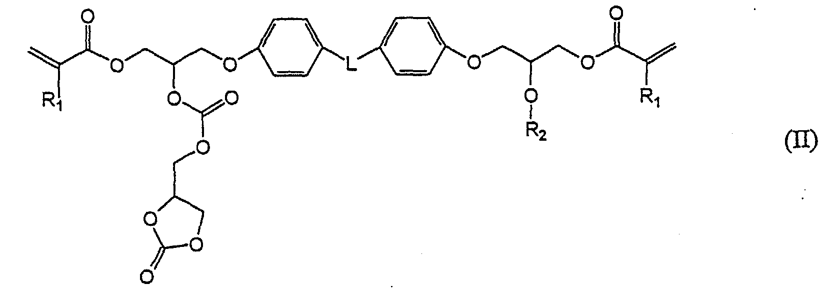 worin unabhängig voneinander
R₁ unabhängig voneinander für Wasserstoff oder Methyl,
R₂ für Wasserstoff oder 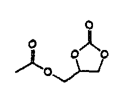 und
L für eine direkte C-C-Bindung, -O-, -S-, -SO₂-, -CO-, einen C₁- bis C₁₅-Alkylenrest, der mit Alkyl, Hydroxyl oder Halogen substituiert sein kann, den Cyclohexylidenrest oder den 3,3,5-Trimethylcyclohexylidenrest steht.

2. Verbindungen gemäß Anspruch 1, dadurch gekennzeichnet, daß in Formel (II)
L für 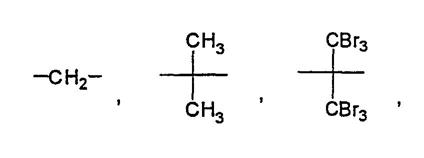 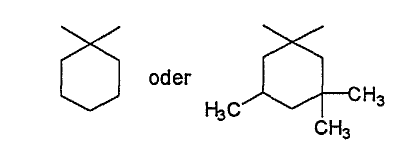 steht.

3. Verbindungen gemäß Ansprüchen 1 und 2 aus der Gruppe von Verbindungen, bestehend aus

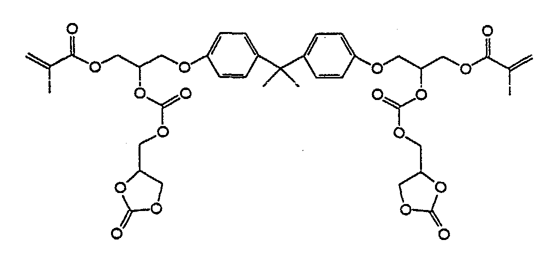

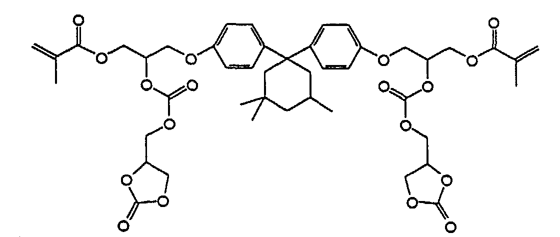

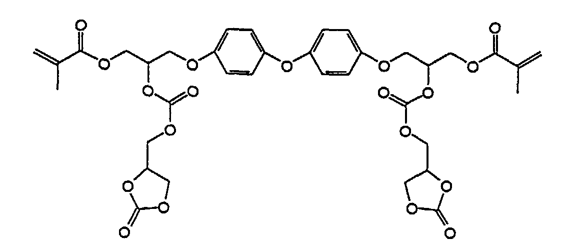

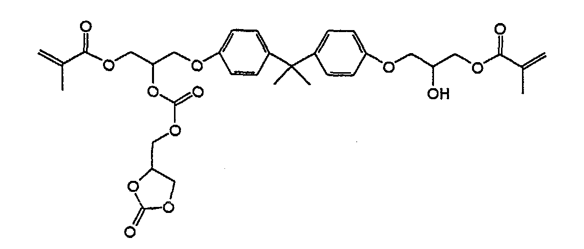

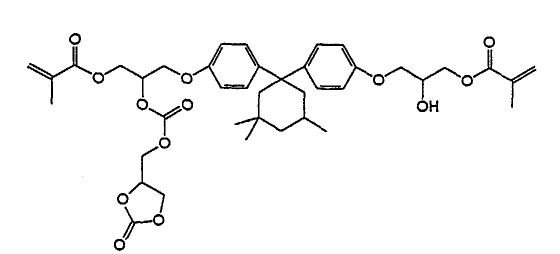

4. Verfahren zur Herstellung von Verbindungen der Formel (II), 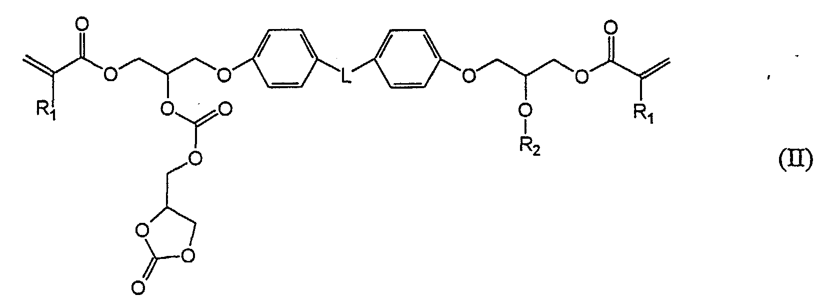 worin unabhängig voneinander
R₁ unabhängig voneinander für Wasserstoff oder Methyl,
R₂ für Wasserstoff oder 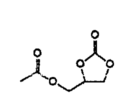 und
L für eine direkte C-C-Bindung, -O-, -S-, -SO₂-, -CO-, einen C₁- bis C₁₅-Alkylenrest, der mit Alkyl, Hydroxyl oder Halogen substituiert sein kann, den Cyclohexylidenrest oder den 3,3,5-Trimethylcyclohexylidenrest steht,
dadurch gekennzeichnet, daß man Verbindungen der Formel (IIa) 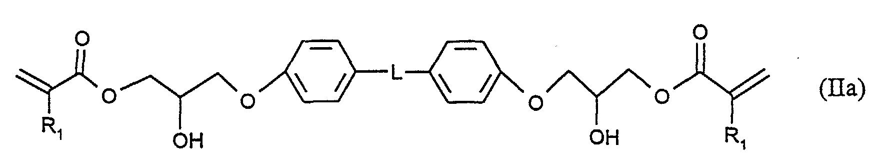 worin
R₁ und L die vorgenannte Bedeutung haben,
mit einem Chlorameisensäureester der Formel (III) 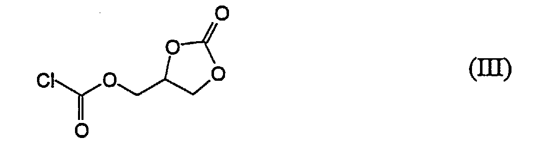 umsetzt.

5. Verfahren gemäß Anspruch 4, dadurch gekennzeichnet, daß in Formel (IIa)
L für 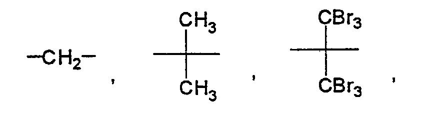 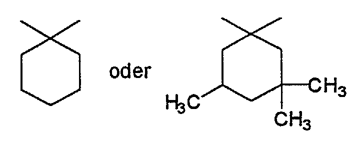 steht.

6. Verwendung von Verbindungen gemäß Ansprüchen 1 bis 3 bei der Herstellung von Restaurationsmaterialien in der Dentaltechnik.

7. Restaurationsmaterialien in der Dentaltechnik, enthaltend Verbindungen gemäß Ansprüchen 1 bis 3.

## Claims

1. Compounds of the formula (II) 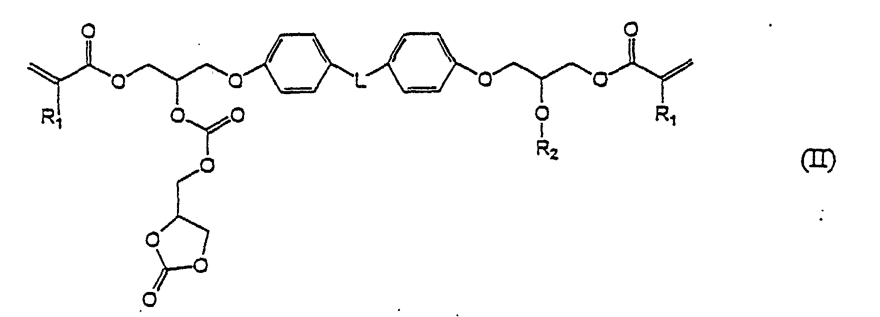 in which
R₁, independently of one another, are hydrogen or methyl,
R₂ is hydrogen or 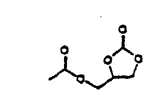 and
L is a direct C-C bond, -O-, -S-, -SO₂-, -CO-, a C₁-C₁₅-alkylene radical which may be substituted by alkyl, hydroxyl or halogen, the cyclohexylidene radical or the 3,3,5-trimethylcyclohexylidene radical.

2. Compounds according to Claim 1, characterized in that, in the formula (II),
L is

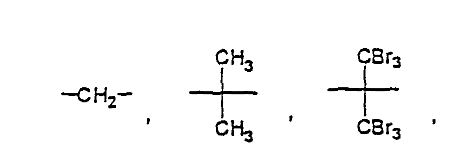

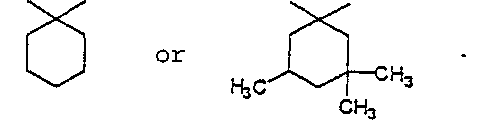

3. Compounds according to Claims 1 and 2 from the group of compounds consisting of

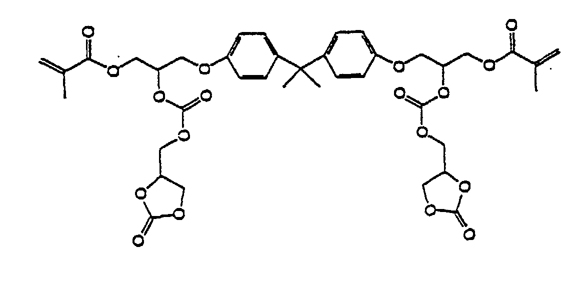

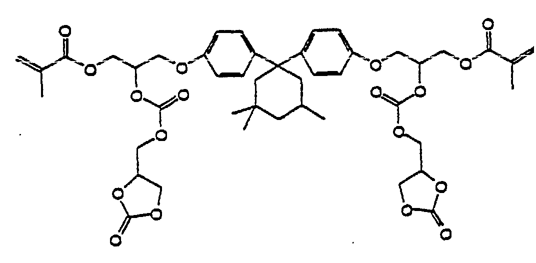

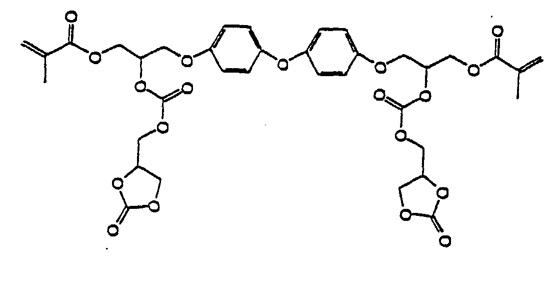

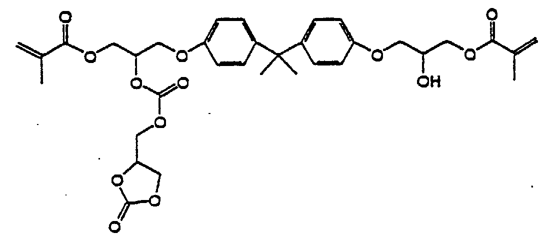

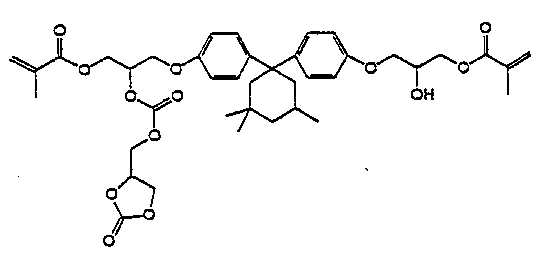

4. Process for the preparation of compounds of the formula (II) 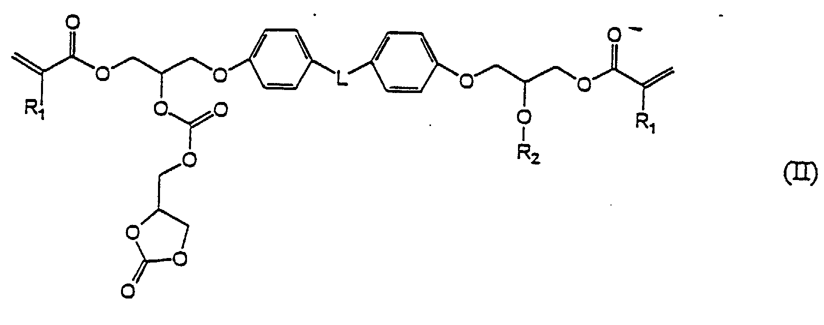 in which
R₁, independently of one another, are each hydrogen or methyl,
R₂ is hydrogen or 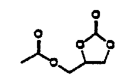 and
L is a direct C-C bond, -O-, -S-, -SO₂-, -CO-, a C₁-C₁₅-alkylene radical which may be substituted by alkyl, hydroxyl or halogen, the cyclohexylidene radical or the 3,3,5-trimethylcyclohexylidene radical,
characterized in that compounds of the formula (IIa) 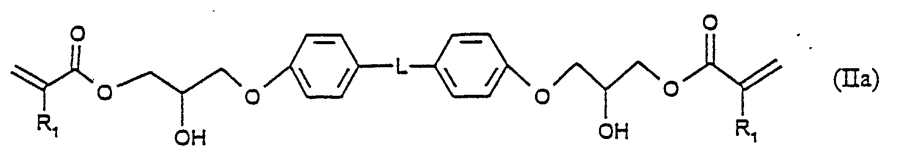 in which
R₁ and L have the above-mentioned meaning,
are reacted with a chloroformic ester of the formula (III)

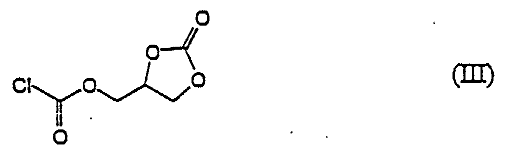

5. Process according to Claim 4, characterized in that, in the formula (IIa),
L is

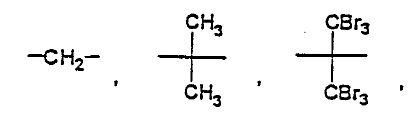

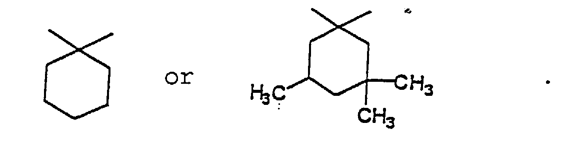

6. Use of compounds according to Claims 1 to 3 in the preparation of restoration materials in dental technology.

7. Restoration materials in dental technology, containing compounds according to Claims 1 to 3.

## Revendications

1. Composés de formule (II) 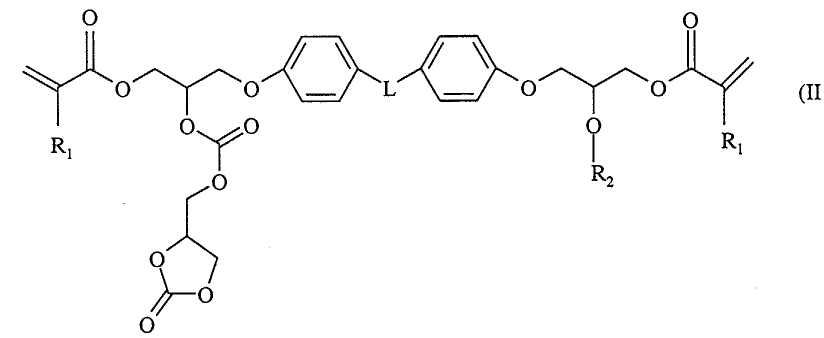 où indépendamment l'un de l'autre
R₁ représente indépendamment l'un de l'autre un hydrogène ou un méthyle,
R₂ représente un hydrogène ou 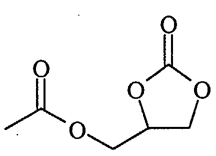 et
L représente une liaison directe C-C-, -O-, -S-, -SO₂-, -CO-, un reste alkylène C₁ à C₁₅, qui peut être substitué par un alkyle, un hydroxyle ou un halogène, le reste cyclohexylidène ou le reste 3,3,5-triméthylcyclohexylidène.

2. Composés selon la revendication 1, caractérisé en ce que dans la formule (II)
L représente

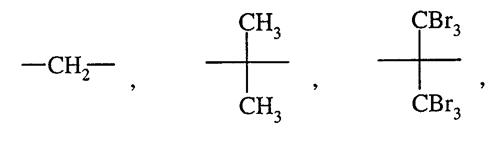

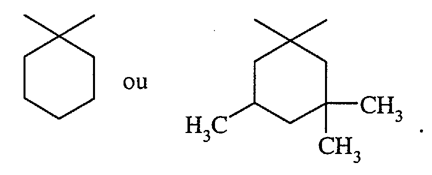

3. Composés selon les revendications 1 et 2 du groupe des composés constitués de

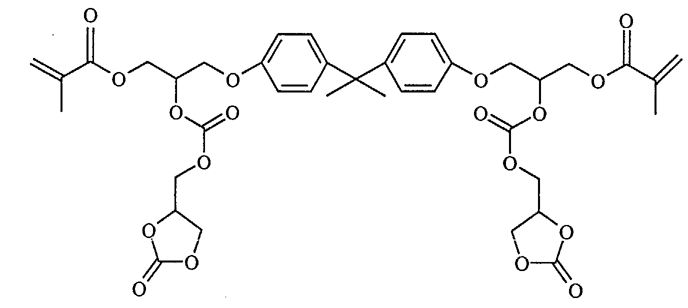

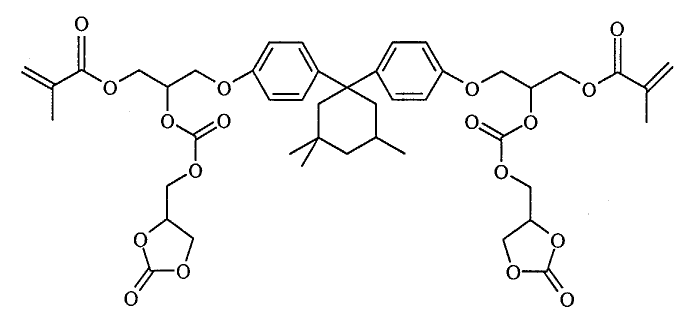

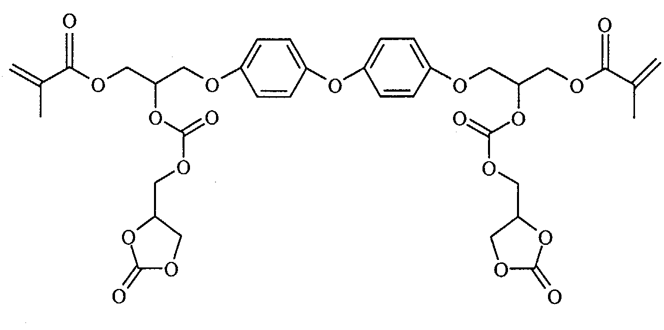

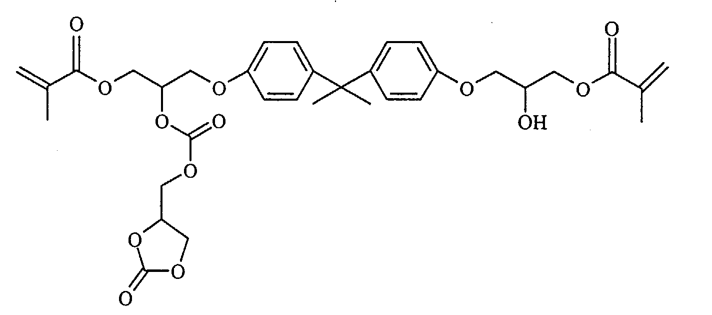

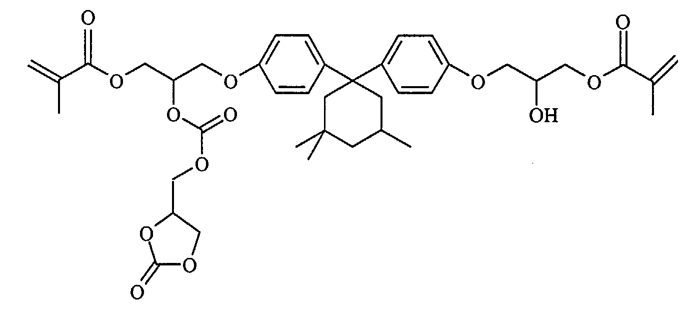

4. Procédé de préparation de composés de formule (II), 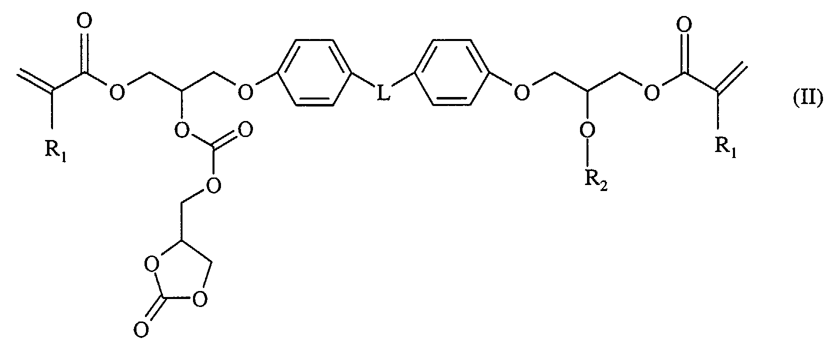 où indépendamment l'un de l'autre
R₁ représente indépendamment l'un de l'autre un hydrogène ou un méthyle,
R₂ représente un hydrogène ou 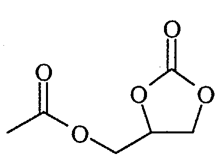 et
L représente une liaison directe C-C-, -O-, -S-, -SO₂-, -CO-, un reste alkylène C₁ à C₁₅, qui peut être substitué par un alkyle, un hydroxyle ou un halogène, le reste cyclohexylidène ou le reste 3,3,5-triméthylcyclohexylidène,
caractérisé en ce qu'on fait réagir des composés de formule (IIa) 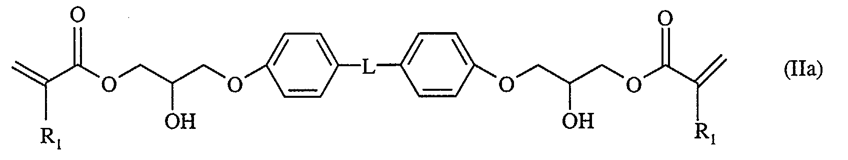 où
R₁ et L ont la signification donnée ci-dessus,
avec un ester d'acide chloroformique de formule (III)

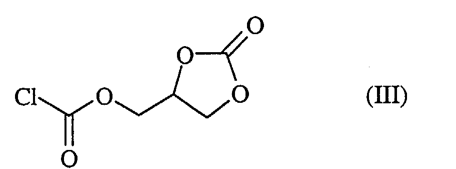

5. Procédé selon la revendication 4 caractérisé en ce que dans la formule (IIa) L représente

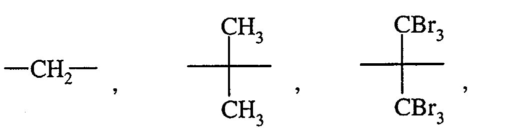

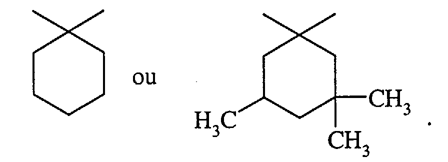

6. Utilisation des composés selon les revendications 1 à 3 pour préparer des matériaux de réparation en technique dentaire.

7. Matériaux de réparation en technique dentaire, contenant des composés selon les revendications 1 à 3.
